(19) Europäisches Patentamt
European Patent Office
Office européen des brevets

(11) **EP 2 296 978 B1**

(12) **EUROPEAN PATENT SPECIFICATION**

(45) Date of publication and mention
of the grant of the patent:
**13.06.2018 Bulletin 2018/24**

(51) Int Cl.:
**B65B 55/10** (2006.01)   **A61L 2/18** (2006.01)
**G01N 25/42** (2006.01)

(21) Application number: **09798202.9**

(22) Date of filing: **06.07.2009**

(86) International application number:
**PCT/SE2009/000355**

(87) International publication number:
**WO 2010/008334 (21.01.2010 Gazette 2010/03)**

(54) **DEVICE FOR CONCENTRATION MEASUREMENTS AND STERILIZATION CHAMBER AND FILLING MACHINE COMPRISING SAID DEVICE**

VORRICHTUNG ZUR KONZENTRATION VON MESSUNGEN SOWIE STERILISIERUNGSKAMMER UND FÜLLMASCHINE MIT BESAGTER VORRICHTUNG

DISPOSITIF POUR DES MESURES DE CONCENTRATION ET CHAMBRE DE STÉRILISATION ET MACHINE DE REMPLISSAGE COMPRENANT LEDIT DISPOSITIF

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **14.07.2008 SE 0801681**

(43) Date of publication of application:
**23.03.2011 Bulletin 2011/12**

(73) Proprietor: **Tetra Laval Holdings & Finance S.A.**
**1009 Pully (CH)**

(72) Inventors:
• **OLANDERS, Pär**
  **S-247 72 Genarp (SE)**
• **ANDERSSON, Jan**
  **S-271 94 Ystad (SE)**
• **SAEIDIHAGHI, Arash**
  **224064 Lund (SE)**

(74) Representative: **Tetra Pak - Patent Attorneys SE**
**AB Tetra Pak**
**Patent Department**
**Ruben Rausings gata**
**221 86 Lund (SE)**

(56) References cited:
| | |
|---|---|
| **EP-A1- 0 818 676** | **WO-A1-03/030950** |
| **WO-A1-2004/054882** | **WO-A1-2008/088248** |
| **WO-A1-2008/088248** | **GB-A- 761 055** |
| **JP-A- 2000 241 375** | **US-A- 4 170 455** |
| **US-A- 5 997 827** | **US-A- 6 096 265** |
| **US-A- 6 096 265** | **US-A1- 2005 013 726** |
| **US-A1- 2006 088 441** | **US-A1- 2006 088 441** |
| **US-A1- 2008 098 799** | **US-A1- 2008 098 799** |
| **US-A1- 2008 264 140** | **US-A1- 2009 087 921** |

• **DATABASE WPI Week 200103, Derwent Publications Ltd., London, GB; AN 2001-018967, XP003026056 & JP 2000 241375 A (TOYOTA JIDOSHA KK) 08 September 2000**

## Description

### TECHNICAL FIELD OF THE INVENTION

The present invention relates to a device for concentration measurements, and a sterilization chamber of a filling machine for producing food packages. The invention also relates to a machine for producing packages comprising such a device.

### BACKGROUND ART

Within the food industry, beverages and other products are often packed in paper or paperboard based packages. Packages intended for liquid food are often produced from a packaging laminate comprising a core layer of paper or paperboard and an outer, liquid-tight layer of thermoplastic material on at least that side of the core layer which will form the inside of the packages. For particularly oxygen sensitive food products, such as fruit juice and cooking oil, the packaging laminate usually further comprises a layer of a gas barrier material. This layer is in most cases an aluminum foil which also enables induction sealing of the packaging laminate.

The packages are often produced in a packaging machine where a web of packaging laminate is formed into a tube which is closed by sealing of the longitudinal edges of the web in an overlapping condition. The longitudinally sealed tube is continuously filled with a product and then transversally sealed, wherein filled "cushions" are formed. The transverse sealing is made along narrow, transverse, mutually spaced apart, sealing zones. After the transverse sealing, the "cushions" are separated from the rest of the tube by incisions in the sealing zones and finally formed into the desired shape.

Nowadays, also so-called carton bottles are frequently occurring packages. In substance, these are composed of a lower part in the form of a sleeve of packaging laminate like the one described above, and an upper part in the form of a plastic top provided with an opening device, such as a cap. The carton bottles are often produced in a packaging machine where sheets, so-called blanks, of packaging laminate are formed into tubes which are closed by sealing of two opposing edges of each sheet in an overlapping condition. Then, each tube is slipped over a respective plastic top and arranged in such a way that a major part of the plastic top protrudes from the tube. After sealing of the top and the tube along a contact surface between them, the package is filled, sealed at the open end of the tube for achieving a sleeve and closing the package, and finally formed into the desired shape.

The manufacturing processes above are well known and will not be described in detail.

To extend the shelf life of the products packed, it is prior known to sterilize the packaging material prior to the filling operation and sometimes also the forming operation. Depending on the desired shelf life and whether the packages are to be distributed and stored in a chilled or ambient environment, different levels of sterilization can be chosen.

A known way of sterilizing packaging material is chemical sterilization. As an example, chemical sterilization can be made by passing the packaging material through a bath of hydrogen peroxide solution and then through a heating chamber. In the heating chamber, hot sterile air is introduced for heating the packaging material to such a degree that the hydrogen peroxide is evaporated and thus removed from the surface of the packaging material.

The above method works excellent when it comes to sterilization of a continuous web of packaging laminate but is obviously less suited for sterilization of open carton bottles prior to filling.

In connection with carton bottles, so-called gas phase sterilization can instead be used. Such sterilization can be made by passing the carton bottles through a preheating chamber for preheating, a gassing chamber containing gaseous hydrogen peroxide and then through a venting chamber. In the venting chamber, the carton bottles are exposed to a flow of hot sterile air for removal of residues of hydrogen peroxide. A gas phase sterilization device adapted for sterilization of carton bottles is known from the Swedish patent number 0203692-9. For proper sterilization of the packaging material, in the peroxide bath case, accurate control of the hydrogen peroxide concentration in the gas mixture inside the heating chamber is crucial. If the concentration is too low, the hydrogen peroxide applied onto the packaging material evaporates too quickly resulting in a risk of insufficient sterilization. On the other hand, if the concentration is too high, the hydrogen peroxide applied onto the packaging material evaporates too slowly resulting in a risk of unallowable peroxide residues on the packaging material leaving the heating chamber. Similarly, in the gas phase sterilization case, accurate control of the hydrogen peroxide concentration in the gas mixture inside the gassing chamber is crucial. If the concentration is too low, there is a risk of insufficient sterilization. Further, if the concentration is too high, there is a risk of too much peroxide on the carton bottles leaving the gassing chamber and, consequently, a risk of unallowable peroxide residues on the carton bottles leaving the venting chamber. In either case, if the peroxide concentration is not within certain limits, the settings of the packaging machine should be changed.

There are many ways of determining a hydrogen peroxide concentration. In a known packaging machine with a hydrogen peroxide bath as described above, the hydrogen peroxide concentration in the gas mixture inside the heating chamber is estimated by the machine control system based on the concentration of hydrogen peroxide in the solution, the consumption of hydrogen peroxide solution and the air mass flow inside the heating chamber. Thus, here the hydrogen peroxide concentration is theoretically calculated from known external parameters. This is a somewhat uncertain determination method and it involves a risk of erroneous concentration determinations and, thus, a risk of false alarms, or the packaging material being insufficiently sterilized or too high levels of residual hydrogen peroxide being left on the packaging material.

In a known packaging machine with a gassing chamber as described above, the hydrogen peroxide concentration in the gas mixture inside the gassing chamber is measured by means of IR-sensors or UV-sensors. However, such sensors are bulky, fragile and cost a lot of money.

A related device for measuring a concentration of hydrogen peroxide is disclosed in WO 2008/088248.

## SUMMARY OF THE INVENTION

It is an object of the present invention to at least partly overcome or mitigate some of the problems of the prior art. This is accomplished by a device according to claim 1, where preferred embodiments are given by the dependent claims.

## BRIEF DESCRIPTION OF THE DRAWINGS

The invention will be described in more detail with reference to the appended schematic drawings, which show examples of presently non-limiting preferred embodiments of the invention, where

Fig. 1 is a schematical drawing of a heating chamber in a filling machine having a device for concentration measurements in accordance with the invention,
Fig. 2 is a cross-sectional view of the device as is shown in Fig. 1,
Fig. 3 is a cross-sectional view of a second embodiment of the device according to Fig. 2 and
Fig. 4 is another schematical drawing of a heating chamber in filling machine, having a device according to the invention.

## DETAILED DESCRIPTION OF PREFERRED EMBODIMENTS

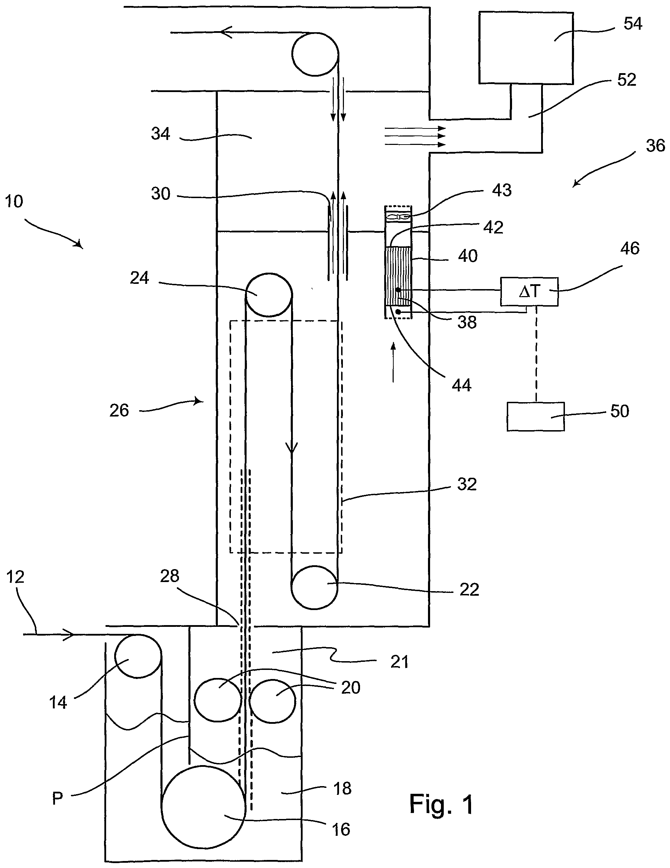

In Fig. 1, a machine 10 working in accordance with a predetermined process for producing packages from a tube (not shown) of packaging laminate is shown. The packaging laminate is of the initially described kind, i.e. it comprises a paper core layer, an aluminum gas barrier layer and outer layers of thermoplastic material, and similar kinds of packaging material are also possible, e.g. with additional layers or without an aluminum layer. As initially described, the tube is formed by sealing of the longitudinal edges of a web 12 of packaging laminate in an overlapping condition. After forming, the tube is filled with the intended product and transversally sealed at regular intervals. The "cushions" thus formed are separated from the rest of the tube and finally formed into the desired shape. Continuous forming, filling and sealing of a tube like this is well-known and will not be described in detail herein.

Prior to tube forming, the web 12 of packaging laminate is sterilized. To this end, the web 12 is directed, by means of directing rollers 14 and 16, through a bath 18 containing a hydrogen peroxide solution. As an example, the bath can be of any of the types described in applicant's Chinese patent applications number 200510069635.8 and 200510133873.0. After leaving the bath 18, the web 12 is passed between two squeegee rollers 20 for evenly spreading out the peroxide solution over the entire width of the web 12 and removing excess peroxide. Then, the web 12, now carrying droplets of hydrogen peroxide solution, is directed, by means of directing rollers 22, 24, through a heating chamber 26 for further hydrogen peroxide removal by evaporation. The web 12 enters the heating chamber 26 through an entrance opening 28 and exits the same through an exit opening 30.

The sterilization or heating chamber 26 comprises a heating arrangement, schematically illustrated by the box with dashed lines and denoted 32 in Fig. 1, for effecting the evaporation of the hydrogen peroxide solution applied onto the web 12. The heating arrangement 32, which, for example, can be of the type described in applicant's Chinese patent application number 200510069630.5, is arranged to introduce hot sterile air into the heating chamber. By hot air is meant air of a temperature that is high enough to perform the evaporation of the sterilization agent used, here hydrogen peroxide. After leaving the heating chamber 26, the web 12 passes an intermediate chamber 34 before the tube forming, filling and sealing takes place. The heating arrangement thus comprises means for supplying air to the heating chamber 26, such as a fan or similar (not shown).

In order to achieve a proper sterilization of the web 12, it is very important that the environment inside the heating chamber is carefully controlled. Specifically, it is desirable to frequently measure the hydrogen peroxide concentration in the gas mixture inside the heating chamber 26, which gas mixture consists of air and gaseous hydrogen peroxide evaporated from the web of packaging laminate when passing through the heating chamber 26. The hydrogen peroxide concentration inside the heating chamber is dependent upon a number of different parameters, such as the hydrogen peroxide concentration in the solution in the bath 18, the consumption of the solution in the bath and the flow of hot sterile air into the chamber. If the hydrogen peroxide concentration inside the heating chamber is too high, there is a risk of unallowable residues of hydrogen peroxide on the web when this leaves the heating chamber. On the other hand, if the hydrogen peroxide concentration inside the heating chamber is too low, there is a risk that the web is inadequately sterilized when it leaves the chamber. If the hydrogen peroxide concentration is not within predetermined limits, the settings of the machine should preferably be adjusted.

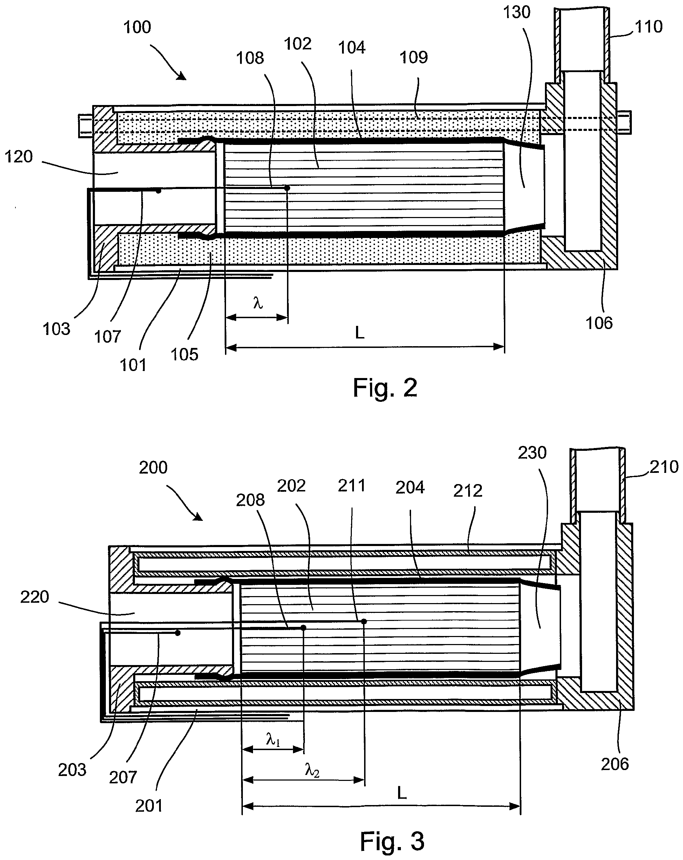

In accordance therewith, the heating chamber 26 further has a device 36, 100, 200 for determining the concentration of hydrogen peroxide in the gas mixture inside the heating chamber 26, one embodiment 100 of said device is shown in detail in Fig. 2. The device 100 comprises a housing 101 accommodating a measuring catalyst means or catalyst 102 in the form of a body of ceramic material dipped in precious metal solution, such as platinum solution. The catalytic body can have an outer shape of a rectangular parallelepiped. Channels with essentially square-shaped cross-sections running side by side in the longitudinal direction of rectangular parallelepiped fill up the entire interior of the catalyst 102. This structure gives the catalyst 102 a lattice-like cross-section in the transverse directions. A circular cross-section or similar of the catalyst body 102 is also possible, as well as channels therein having circular cross-sections or similar. The catalyst body 102 is in one embodiment manufactured by a material having good thermal conduction, such as a metal or similar. This reduces the temperature gradients in the x-direction and improves the reliability of the measurements.

The catalyst 102 is surrounded by the housing 101, e.g. made of stainless steel or similar. At the upstream end of the housing 101, a first end piece 103 is mounted. This first end piece 103 is in one embodiment manufactured of an insulating material, such as a polymer or similar. The catalyst 102 is in Fig. 2 fixed to the first end piece 103 by a shrink hose 104, which encompasses the catalyst body 102 and ends at the outlet of the catalyst body. The shrink hose 104 separates surrounding insulation 105 from being in contact with the catalyst body 102, and also allows the catalyst body 102 to be fixed at a certain distance from the first end piece 103. The housing 101 is at a downstream end attached to a second end piece 106, which in turn is connected to a pipe 110 that is arranged to direct exhausts out of the device 100. It also possible to have an air gap between the outer housing 101 and the catalyst 102, for insulation purposes.

A first thermometer 107 is arranged at a certain distance upstream of the entrance to the catalyst body 102. A second thermometer 108 is arranged at a certain distance into the catalyst body 102, and is in one embodiment inserted in a channel of the catalyst 102, either from the upstream or downstream end of the catalyst. The first end piece 103, the housing 101 and the second end piece 106 are in the shown embodiment held together by a threaded rod and screw nuts 109, indicated by dashed lines in Fig. 2. In one embodiment, a fan or similar (only shown in Fig. 1) is mounted in the device 100, either at the upstream end or the downstream end thereof, for driving a flow through the device 100. In another embodiment, the flow is driven by a pressure difference over the inlet and the outlet.

The first thermometer 107 is in the shown embodiment located in the inlet 120 of the device 100, and thus measures the temperature of the gas going into the device. This thermometer can hence also be located outside of the device 100, but preferably in close proximity to the device 100. The second thermometer 108 is located a certain distance $\lambda$ into the catalyst body 102 having a total length of L. The specific distance $\lambda$ should be within a certain range, given by initial calibration measurements. In one embodiment, the distance $\lambda$ is about 15-45 % of the total length L, in another embodiment, the distance $\lambda$ is about 20-40% of the total length L, and in yet another embodiment, the distance $\lambda$ is about 30% of the total length L. In one embodiment, the total length of the catalyst body 102 is about 100 mm.

The optimum location for the second thermometer 108 depends on the mass flow, and it is beneficial if substantially all hydrogen peroxide, is oxidized at the measurement point. The steps for choosing an optimum point are as follows: a) direct a flow of hydrogen peroxide, having an average concentration, through the catalyst 102 at a first minimum allowed mass flow, b) measure the longitudinal location of the maximum temperature in the catalyst 102, measuring along the central axis of the catalyst 102, c) increase the mass flow of the hydrogen peroxide through the catalyst 102 to a maximum allowed level, without changing the concentration, d) measure the longitudinal location of the maximum temperature in the catalyst 102, measuring along the central axis of the catalyst 102, e) calculate the optimum longitudinal location as the average of the above two longitudinal locations.

The specific arrangement of the device 100, in which the shrink hose 104 holds the catalyst body 102 almost floating inside the housing 101, reduces the heat losses to the surrounding. The location of the second thermometer 108 inside the catalyst body 102 removes large heat loss gradients around the measurement point. This improves the accuracy.

The chosen point for the second thermometer 108 can be at the center of the range in which the ingoing hydrogen peroxide, at minimum and maximum mass flow, is completely decomposed. Calibration measurements can be made in order for this point to be found. The accuracy of the concentration measurement device 100 is sufficient in this region, for mass flows within the minimum and maximum level. When the catalyst 102 gets older, the optimal point for measuring the second temperature is moved towards the outlet. For this reason, it is beneficial if the location of the second thermometer 108 is offset slightly towards the outlet 130, in relation to the optimum placement for compensating for the mass flow.

A second embodiment of the device 200 according to the invention is shown in Fig. 3. The device has a housing 201 being attached to a first end piece 203 at an upstream end and a second end piece 206 at a downstream end. A catalyst 202 is arranged centrally inside the housing, generally between the first and second end pieces 203, 206. The catalyst 202 can be attached to the first end piece 203 with a shrink hose 204 or similar, and in one embodiment the catalyst 202 is arranged at a certain distance from the first end piece 203.

The catalyst is surrounded by an outer insulator 212, in the shown embodiment comprising two concentrically arranged containers being closed off at either end. The distance between the containers may be kept at a vacuum, or can be filled with a gas of low thermal conductivity. The insulator 212 is in one embodiment provided with polished surfaces, in order to reduce the heat radiation. In one embodiment, the housing can be omitted and the insulator 212 can be used as the housing.

In the shown embodiment, the device 200 is equipped with three thermometers, where the first thermometer 207 measures a temperature T0 in the inlet of the device 200, the second thermometer measures a temperature T1 at a distance $\lambda_1$ inside the catalyst 202, and the third thermometer 211 measures a temperature T2 at a distance $\lambda_2$ inside the catalyst 202.

The device 36, 100, 200 further has means 50 for calculating a concentration based on the temperature measurements from the at least two thermometers 107, 108. This means can also comprise a memory for storing calibrated values, or a look-up table. This means can be a simple processor or similar device that is able to calculate the difference between the first and the second temperature, and then, using stored values, can calculate the concentration.

In one embodiment, the higher pressure inside the heating chamber 26, see Fig. 1, forces the gas mixture of hydrogen peroxide and air through the exit opening 30 and through the housing 40, and thus also through the catalyst 38, 100. Essentially no gas mixture leaves the heating chamber 26 through the entrance opening 28 because of the construction of the bath 18. When the gas mixture passes through the catalyst 38, the hydrogen peroxide is spontaneously decomposed into water and oxygen in an exothermic reaction according to the formula below:

$$H_2O_2(g) \rightarrow H_2O(g) + 1/2 O_2(g) \qquad \Delta H_{Dec} = 23.44 \, kcal/mol$$

The concentration measurement device is equipped with at least two thermometers, the first thermometer at a certain distance from the inlet and the second at a certain distance into the catalyst. The second thermometer is in one embodiment very thin and can be inserted into one of the channels in the catalytic body 102.

The flow through the catalyst is normally driven by a pressure difference between the inlet, located inside the heating chamber 26, and the outlet, located inside an outlet of the filling machine, where the pressure is significantly lower than inside the heating chamber. The flow through the catalyst can also be provided by a fan 43 or similar, see Fig. 1, which can be electrically driven or by an equivalent means.

Because of the arrangement of the catalyst 102 inside the housing 101 with insulation 105 and within the heating chamber 26, most of the heat generated due to the decomposition of the hydrogen peroxide concentration is kept within the catalyst 102 resulting in a temperature rise therein.

The equation that governs the reactions inside the catalyst is $\dot{m} \cdot c \cdot h = \dot{m} \cdot c_p \cdot \Delta T + Q_{hl}$ where $\dot{m}$ is the mass flow, $c$ is the concentration, h is the enthalpy, $c_p$ is the specific heat and $Q_{hl}$ are the heat losses. If the catalyst is large, the degree of breakdown of the hydrogen peroxide is high. By minimizing the heat losses, through the use of proper insulation, the equation becomes $\dot{m} \cdot c \cdot h = \dot{m} \cdot c_p \cdot \Delta T$ which can be shortened into $c \cdot h = c_p \cdot \Delta T$, where the mass flow no longer influences the concentration. Experiments have shown that this is only true at a certain length interval in the catalyst, namely where substantially all hydrogen peroxide has been decomposed, and the heat losses have not lowered the temperature significantly. The exact relationship between the temperature difference and the concentration must be derived through calibrations. Such reference measurements can e.g. be made with a mass flow meter, a calorimetric mass flow meter or similar accurate reference mass flow meter, and a UV concentration sensor or similar accurate concentration measurement means.

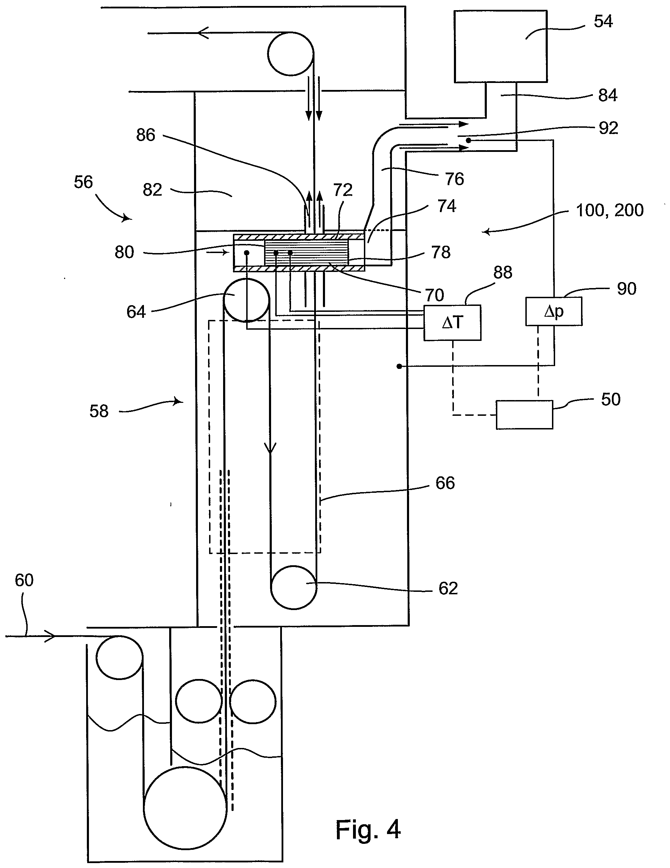

For obvious reasons, it is not desirable to release gaseous hydrogen peroxide into the open. For this reason, an outlet pipe 52 of the intermediate chamber 34 is connected to a main catalyst 54 (schematically illustrated) for hydrogen peroxide decomposition prior to discharge, see Fig. 4. The gas mixture inside the intermediate chamber 34 is driven through the outlet pipe 52 and the main catalyst 54 by a higher pressure being maintained inside the intermediate chamber 34 than out in the open, or by the fan or similar. The main catalyst 54 is a larger version of the catalyst 38 and will not be further described herein.

In Fig. 4, a machine 56 working in accordance with a predetermined process for producing packages from a tube of packaging laminate is shown. The machine 56 is, in most aspects, identical to the machine shown in Fig. 1 and described above. Below, as far as possible, only the parts of the machine 56 differing from the corresponding parts of the machine 10 will be described.

The machine 56 comprises a heating chamber 58 through which a web 60 of packaging laminate is directed, by means of directing rollers 62, 64, for hydrogen peroxide removal by evaporation. The heating chamber 58 has a heating arrangement 66 for effecting the evaporation and a device 100, 200 for determining the concentration of hydrogen peroxide in the gas mixture inside the heating chamber 58. The device 100, 200 comprises a catalyst 70 of the same type as the catalyst 38 described above. The catalyst 70 is surrounded by an insulated housing 72, e.g. made of a mixture of glass and Teflon®, foamed silicone or foamed Teflon® or similar, having poor heat conductivity both in the radial direction and the longitudinal direction of the housing. As apparent from the figures, the catalyst and, thus, the insulated housing, are arranged essentially vertically in Fig. 1 and essentially horizontally in Fig. 2. Both arrangements work equally well and can be chosen in dependence upon the physical properties of the rest of the machine. The device 200 can also have an identical construction to the device 100.

The catalyst 70 and the insulating housing 72 are arranged inside the heating chamber 58. The housing 72 has an outlet 74 which is connected to a pipe 76 of preferably stainless steel leading out of the heating chamber 58 such that an outlet 78 of the catalyst 70 is directly connected to the outside of the heating chamber, whereas an inlet 80 of the catalyst 70 is directly connected to the inside of the heating chamber 58. The machine 56 further comprises an intermediate chamber 82 similar to the intermediate chamber 34 of the above described machine 10. The pipe 76 leads through the intermediate chamber 82 and well into an outlet pipe 84 of the same.

Just as described above, the gas mixture inside the heating chamber is forced through, not only the housing 72 and the catalyst 70, but also an exit opening 86 of the heating chamber 58. The hydrogen peroxide in the gas mixture is, at least partly, decomposed into water and oxygen inside the catalyst 70, whereby heat is generated causing a temperature rise therein. Further, as discussed above, there is a pressure drop across the housing 72. If no pressure difference is present over the catalyst, a separate means can be arranged for driving a flow through the catalyst body 70, as is shown in Fig. 1.

If the temperature rise in the catalyst 70 is known, the concentration of hydrogen peroxide in the gas mixture inside the heating chamber 58 can be readily calculated by the above formula. In accordance therewith, the device 100, 200 further comprises thermometers 88 for measuring a first temperature inside the housing at the inlet 80 of the catalyst 70 and a second temperature inside the catalyst body 70. In order to obtain an increased pressure difference between the first and the second pressures, and, thus, in the end, a greater mass flow, the pipe 76 can be located in the outlet pipe 84 of the intermediate chamber 82.

The above formula can also be written

$$C_0 = \frac{c_p}{\Delta h} \cdot \Delta T = const \cdot \Delta T \, .$$

At 100 °C and a concentration of about 12 000 ppm, the above constant is given by the following expression:

$$\frac{c_p}{\Delta h} = (1.04 \, \text{kJ/kg°K}) / (2887 \, \text{kJ/kg}) = 358 \cdot 10^{-6} \, \left[ 1/°\text{K} \right].$$

The concentration can then be calculated if the temperature difference is known. In the embodiment shown in Fig. 3, two temperature differences can be measured, the first between the first thermometer 207 and the second thermometer 208, labeled $\Delta T1$, and the second between the first thermometer 207 and the third thermometer 211, labeled $\Delta T2$. It has been shown that accurate measurements can be achieved, independent of concentration and mass flow, if the locations of the thermometers are chosen carefully. In one embodiment, the second thermometer 208 is located about 20 mm into the catalytic element 102, as measured from the upstream end, and the second thermometer 211 is located about 50 mm into the catalytic element 102. By calculating the concentration using both the temperature differences, $\Delta T1$ and $\Delta T2$, and using the maximum value, accurate values can be calculated according to

$$C_0 = 358 \cdot 10^{-6} \cdot \max(\Delta T1, \Delta T2)$$

The two temperature differences $\Delta T1$ and $\Delta T2$ can also be used for self-diagnostics. It has been shown that the below calculation D is a good indication of the status of the catalytic element 102:

$$D = \frac{\Delta T2 - \Delta T1}{\Delta T2}$$

When the catalytic element is in good order, the calculation D should be below 0.05. Other values of D are of course possible, depending on the required accuracy of the concentration sensor 100, 200.

The above-described embodiments should only be seen as examples. A person skilled in the art realizes that the embodiments discussed can be modified and varied in a number of ways without deviating from the inventive conception.

As an example, the invention is not limited to sterilization by means of a bath containing hydrogen peroxide but could also be used in connection with other techniques for hydrogen peroxide application, such as gassing, condensing or spraying.

Further, sterilization of a continuous web of packaging laminate, with improved control of the hydrogen peroxide concentration in the heating chamber, has been described above. Naturally, the invention is equally applicable for sterilization of partly formed packages, such as carton-bottles with an open end as initially described or plastic bottles of different kinds, with improved control of the hydrogen peroxide concentration in the gassing chamber. In such an embodiment, the gassing chamber should preferably comprise conveying means, such as a conveyor belt, for conveying the partly formed packages through the gassing chamber.

A thermometer as stated in the application can be any device which is used for measuring a temperature, such as a thermocouple, a thermistor, a bi-metal thermometer, an electrical resistance thermometer, an infrared thermometer etc. In the same way, the catalyst could be made of any suitable materials and have any suitable design, the above compositions and constructions just being examples. Of course, this is also true for the housing, which can be constructed in a large number of different ways. As an example, a tubular insulating housing could be made of two concentric plastic tubes of different diameters arranged with an intermediate air gap.

It should be stressed that a description of details not relevant to the invention has been omitted and that the figures are not drawn according to scale.

Further, it should be pointed out that the term packaging material as used herein is a generic term for all the matter required to form a specific package, for example packaging laminate, plastic top, etc.

**Claims**

1. Device (36, 100, 200) for measuring a concentration of hydrogen peroxide, said device comprising a catalyst (102, 202) having an inlet (120, 220) and an outlet (130, 230), a thermometer (107, 207) being arranged to measure the ambient temperature in the vicinity of the inlet (120, 220), means (43) for driving a flow through the catalyst, **characterized by** having a second thermometer (108, 208; 211) mounted within a channel between the inlet (120, 220) and the outlet (130, 230) of the catalyst (102, 202), and means (50) for calculating a concentration from a difference between a first and second temperature, as measured by said first (107, 207) and second thermometer (108, 208; 211) respectively.

2. Device (36, 100, 200) according to claim 1, further comprising a housing (101) surrounding the catalyst (102), which guides the flow towards the inlet (120) and/or out from the outlet (130).

3. Device (36, 100, 200) according to claim 1, wherein the means for driving the flow is a pressure difference between the inlet (120) and the outlet (130) of the catalyst (102).

4. Device (36, 100, 200) according to claim 2, further having insulation (105) between the housing (101) and the catalyst (102).

5. Device (36, 100, 200) according to claim 2, further having an air gap between the housing (101) and the catalyst (102) for insulation.

6. Device (36, 100, 200) according to claim 2, comprising at the inlet (120) a first end piece (103) of an insulating material to which the catalyst (102) and/or the housing (101) is fixed.

7. Device (36, 100, 200) according to claim 6, wherein the catalyst (102) is connected to the first end piece (103) by a shrink hose (104), said catalyst (102) being arranged at a slight distance from the first end piece (103).

8. Device (36, 100, 200) according to claim 6, wherein the entire catalyst (102) is covered by the shrink hose (103), said shrink hose (103) extending from the outlet (130) of the catalyst (102) and towards the end of the housing (101).

9. Device (36, 100, 200) according to claim 1, wherein the means for driving the flow through the catalyst (102) is a fan (43) or similar.

10. Device (36, 100, 200) according to claim 1, wherein the second thermometer (108) is mounted at a distance from the inlet of the catalyst (102) being in the range of 20-40% of the total length of the catalyst (102).

11. Device (26, 100, 200) according to claim 1, wherein the location of the second thermometer (108) is chosen as the average of the longitudinal location for maximum temperature in the catalyst (1 02) for minimum allowed mass flow, and of the longitudinal location for maximum temperature and maximum allowed mass flow, as measured along the central axis of the catalyst (1 02), for an average concentration.

12. Device according to claim 1, wherein two thermometers (208, 211) are arranged inside the catalyst (202) for measuring two temperatures (T1, T2) at different distances from the inlet of the catalyst (202), and the concentration is calculated from the maximum of the temperature difference between the inlet temperature (TO) and the two temperatures (T1, T2), according to C = const. *max (T1-TO, T2-TO).

13. Device according to claim 12, wherein the two temperatures (T1, T2), measured inside the catalytic element (202) by the second and third thermometers (208, 211), are used for diagnostics of the catalytic element (202), in that the expression (T2-T1)/T2 should be below a certain predetermined level for acceptable measurements.

14. Sterilization chamber (26, 58) for a filling machine for the production of food packages, comprising a device (36, 100, 200) according to claim 1.

15. Filling machine for the production of food packages, comprising a sterilization chamber (26, 58) with a device (36, 100, 200) according to claim 1.


**Patentansprüche**

1. Vorrichtung (36, 100, 200) zum Messen einer Konzentration von Wasserstoffperoxid, wobei die Vorrichtung einen Katalysator (102, 202) mit einem Einlass (120, 220) und einem Auslass (130, 230), ein Thermometer (107, 207), das zum Messen der Umgebungstemperatur in der Nähe des Einlasses (120, 220) angeordnet ist, und Mittel (43) zum Antreiben eines Flusses durch den Katalysator umfasst, **dadurch gekennzeichnet, dass** sie ein zweites Thermometer (108, 208; 211), das innerhalb eines Kanals zwischen dem Einlass (120, 220) und dem Auslass (130, 230) des Katalysators (102, 202) montiert ist, und Mittel (50) zum Berechnen einer Konzentration aus einem Unterschied zwischen einer ersten und einer zweiten Temperatur, wie durch das erste (107, 207) bzw. das zweite Thermometer (108, 208; 211) gemessen, umfasst.

2. Vorrichtung (36, 100, 200) nach Anspruch 1, ferner umfassend ein Gehäuse (101), das den Katalysator (102) umgibt und den Fluss zum Einlass (120) und/oder aus dem Auslass (130) führt.

3. Vorrichtung (36, 100, 200) nach Anspruch 1, wobei das Mittel zum Antreiben des Flusses ein Druckunterschied zwischen dem Einlass (120) und dem Auslass (130) des Katalysators (102) ist.

4. Vorrichtung (36, 100, 200) nach Anspruch 2, ferner aufweisend eine Isolierung (105) zwischen dem Gehäuse (101) und dem Katalysator (102).

5. Vorrichtung (36, 100, 200) nach Anspruch 2, ferner aufweisend einen Luftspalt zwischen dem Gehäuse (101) und dem Katalysator (102) zur Isolierung.

6. Vorrichtung (36, 100, 200) nach Anspruch 2, umfassend am Einlass (120) ein erstes Endstück (103) eines Isoliermaterials, an dem der Katalysator (102) und/oder das Gehäuse (101) fixiert ist/sind.

7. Vorrichtung (36, 100, 200) nach Anspruch 6, wobei der Katalysator (102) durch einen Schrumpfschlauch (104) mit dem ersten Endstück (103) verbunden ist, und der Katalysator (102) in geringem Abstand vom ersten Endstück (103) angeordnet ist.

8. Vorrichtung (36, 100, 200) nach Anspruch 6, wobei der ganze Katalysator (102) durch den Schrumpfschlauch (103) abgedeckt ist, wobei sich der Schrumpfschlauch (103) vom Auslass (130) des Katalysators (102) und zum Ende des Gehäuses (101) erstreckt.

9. Vorrichtung (36, 100, 200) nach Anspruch 1, wobei das Mittel zum Antreiben des Flusses durch den Katalysator (102) ein Gebläse (43) oder dergleichen ist.

10. Vorrichtung (36, 100, 200) nach Anspruch 1, wobei das zweite Thermometer (108) in einem Abstand vom Einlass des Katalysators (102) montiert ist, der im Bereich von 20 bis 40 % der Gesamtlänge des Katalysators (102) liegt.

11. Vorrichtung (26, 100, 200) nach Anspruch 1, wobei die Position des zweiten Thermometers (108) als der Durchschnitt der Längsposition für maximale Temperatur im Katalysator (102) für minimal zulässigen Massendurchfluss und der Längsposition für maximale Temperatur und maximal zulässigen Massendurchfluss, wie entlang einer Mittelachse des Katalysators (102) gemessen, für eine durchschnittliche Konzentration gewählt ist.

12. Vorrichtung nach Anspruch 1, wobei zwei Thermometer (208, 211) innerhalb des Katalysators (202) zum Messen zweier Temperaturen (T1, T2) in verschiedenen Abständen vom Einlass des Katalysators (202) angeordnet sind, und die Konzentration aus dem Maximum des Temperaturunterschieds zwischen der Einlasstemperatur (TO) und den zwei Temperaturen (T1, T2) gemäß C = const. * max. (T1 - TO, T2 - TO) berechnet wird.

13. Vorrichtung nach Anspruch 12, wobei die zwei Temperaturen (T1, T2), die innerhalb des katalytischen Elements (202) durch die zweiten und dritten Thermometer (208, 211) gemessen werden, zur Diagnose des katalytischen Elements (202) verwendet werden, insofern als der Ausdruck (T2 - T1)/T2 unter einer bestimmten vorgegebenen Schwelle für annehmbare Messungen sein sollte.

14. Sterlisationskammer (26, 58) für eine Füllmaschine zur Herstellung von Lebensmittelpackungen, umfassend eine Vorrichtung (36, 100, 200) nach Anspruch 1.

15. Füllmaschine zur Herstellung von Lebensmittelpackungen, umfassend eine Sterlisationskammer (26, 58) mit einer Vorrichtung (36, 100, 200) nach Anspruch 1.

## Revendications

1. Dispositif (36, 100, 200) de mesure d'une concentration de peroxyde d'hydrogène, ledit dispositif comprenant un catalyseur (102, 202) doté d'une entrée (120, 220) et d'une sortie (130, 230), un thermomètre (107, 207) agencé pour mesurer la température ambiante au voisinage de l'entrée (120, 220), un moyen (43) d'entraînement d'un écoulement à travers le catalyseur, **caractérisé en ce qu'**il est doté d'un second thermomètre (108, 208 ; 211) monté dans un canal entre l'entrée (128, 220) et la sortie (130, 230) du catalyseur (102, 202), et un moyen (50) de calcul d'une concentration à partir d'une différence entre une première et une seconde température, mesurées par lesdits premier (107, 207) et second thermomètres (108, 208 ; 211) respectivement.

2. Dispositif (36, 100, 200) selon la revendication 1, comprenant en outre un boîtier (101) autour du catalyseur (102), lequel guide l'écoulement vers l'entrée (120) et/ou en dehors de la sortie (130).

3. Dispositif (36, 100, 200) selon la revendication 1, dans lequel le moyen d'entraînement de l'écoulement est une différence de pression entre l'entrée (120) et la sortie (130) du catalyseur (102).

4. Dispositif (36, 100, 200) selon la revendication 2, doté en outre d'une isolation (105) entre le boîtier (101) et le catalyseur (102).

5. Dispositif (36, 100, 200) selon la revendication 2, doté en outre d'un espace d'air entre le boîtier (101) et le catalyseur (102) à des fins d'isolation.

6. Dispositif (36, 100, 200) selon la revendication 2, comprenant à l'entrée (120) un premier embout (103) d'un matériau isolant auquel le catalyseur (102) et/ou le boîtier (101) est fixé.

7. Dispositif (36, 100, 200) selon la revendication 6, dans lequel le catalyseur (102) est connecté au premier embout (103) par une gaine thermo-rétractable (104), ledit catalyseur (102) étant agencé à une légère distance du premier embout (103).

8. Dispositif (36, 100, 200) selon la revendication 6, dans lequel la totalité du catalyseur (102) est couverte par le gaine thermo-rétractable (103), ladite gaine thermo-rétractable (103) s'étendant depuis la sortie (130) du catalyseur (102) et vers l'extrémité du boîtier (101).

9. Dispositif (36, 100, 200) selon la revendication 1, dans lequel le moyen d'entraînement de l'écoulement à travers le catalyseur (102) est un ventilateur (43) ou moyen similaire.

10. Dispositif (36, 100, 200) selon la revendication 1, dans lequel le second thermomètre (108) est monté à une certaine distance de l'entrée du catalyseur (102) comprise dans la plage de 20 à 40 % de la longueur totale du catalyseur (102).

11. Dispositif (26, 100, 200) selon la revendication 1, dans lequel l'emplacement du second thermomètre (108) est choisi comme la moyenne de l'emplacement longitudinal pour une température maximum dans le catalyseur (102) pour un écoulement massique minimum autorisé, et de l'emplacement longitudinal pour une température maximum et un écoulement massique maximum autorisé, mesurés le long de l'axe central du catalyseur (102), pour une concentration moyenne.

12. Dispositif selon la revendication 1, dans lequel deux thermomètres (208, 211) sont agencés à l'intérieur du catalyseur (202) pour mesurer deux températures (T1, T2) à des distances différentes de l'entrée du catalyseur (202), et la concentration est calculée à partir du maximum de la différence de températures entre la température d'entrée (TO) et les deux températures (TI, T2), en fonction de C = const.* max (T1-TO, T2-TO).

13. Dispositif selon la revendication 12, dans lequel les deux températures (T1, T2), mesurées à l'intérieur de l'élément catalytique (202) par les deuxième et troisième thermomètres (208, 211), servent au diagnostic de l'élément catalytique (202), en ce que l'expression (T2-T1)/T2 doit être inférieure à un certain niveau prédéterminé pour des mesures acceptables.

14. Chambre de stérilisation (26, 58) pour une machine de remplissage pour la production de paquets alimentaires, comprenant un dispositif (36, 100, 200) selon la revendication 1.

15. Machine de remplissage pour la production de paquets alimentaires, comprenant une chambre de stérilisation (26, 58) dotée d'un dispositif (36, 100, 200) selon la revendication 1.

Fig. 1

Fig. 2

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- SE 02036929
- WO 2008088248 A
- CN 200510069635
- CN 200510133873
- CN 200510069630